# EUROPÄISCHE PATENTSCHRIFT

(11) **EP 0 491 000 B1**
(45) Veröffentlichungstag und Bekanntmachung des Hinweises auf die Patenterteilung: **02.11.1994**
(21) Anmeldenummer: 90914699.5
(22) Anmeldetag: 04.09.1990
(51) Int. Cl.: G01N 1/28, G01N 33/00

(54) **VERFAHREN UND EINRICHTUNG FÜR DIE VORBEREITUNG EINES GASGEMISCHES ZUR ANALYSE UND ANWENDUNG DES VERFAHRENS**
PROCESS AND DEVICE FOR PREPARING A GAS MIXTURE FOR ANALYSIS, AND APPLICATION OF THE PROCESS
PROCEDE ET DISPOSITIF POUR LA PREPARATION D'UN MELANGE GAZEUX A DES FINS D'ANALYSE ET APPLICATION DU PROCEDE

(30) Priorität: 04.09.1989 CH 3203/89
(43) Veröffentlichungstag der Anmeldung: 24.06.1992
(73) Patentinhaber: LÖBACH, Ernst, FL-9492 Eschen (LI)
(72) Erfinder: LÖBACH, Ernst, FL-9492 Eschen (LI)
(74) Vertreter: Schaefer, Konrad, Dipl.-Phys.
(86) Internationale Anmeldenummer: EP9001479
(87) Internationale Veröffentlichungsnummer: WO9103719

(56) Entgegenhaltungen:
- FR-E- 1 203 026
- US-A- 4 327 575

## Beschreibung

Die Erfindung bezieht sich auf ein Verfahren zur Bestimmung des Isotopenverhältnisses im ausgeatmeten CO2 einer Atemprobe sowie auf eine Einrichtung zur Durchführung des Verfahrens.

Die Analyse eines Gasgemisches wird dadurch erleichtert, daß es vor der eigentlichen Analyse in verschiedene Fraktionen aufgetrennt wird. Zum Beispiel wird eine Atemluftprobe für die massenspektroskopische Analyse vorbereitet, indem die Person, deren Alveolarluft, also Luft aus der eigentlichen Lunge, untersucht werden soll, einen ungefähr 1 l fassenden Sammelbeutel aufbläst.

Die Atemluftprobe muß vor dem Einlaß in die Ionenquelle des Massenspektrometers physikalisch aufbereitet werden. Dies erfolgt bisher zum Beipiel mit Hilfe eines Kühlfallensystems. Die zu analysierende Atemluft gelangt zunächst in eine mit gefrorenem Methanol gekühlte Falle, in welcher der Wasserdampf ausgefroren wird, danach in eine zweite mit flüssigem Stickstoff gekühlte Falle, in welcher das Kohlendioxid ausgefroren wird. An dieser Stelle werden die nicht ausgefrorenen Gaskomponenten, also insbesondere Stickstoff, Sauerstoff und Edelgase mit Hilfe einer Vakuumpumpe entfernt. Darauf wird die zweite Kühlfalle erwärmt, damit das gefrorene Kohlendioxid verdampfen kann, das nun über ein Einlaßsystem in die Ionenquelle des Massenspektrometers eingelassen wird.

Das geschilderte Verfahren der Probennahme, Speicherung und Aufbereitung hat insbesondere für die Routineanwendung im großen Maßstab erhebliche Nachteile:
- Die Probennahmeprozedur hat ein erhebliches Risiko der Fehlmanipulation.
- Es wird nur eine kleine Luftmenge, die den Bruchteil eines Atemstoßes darstellt, willkürlich herausgegriffen. Das markierte Kohlendioxid fällt aber in der Lunge nicht unbedingt räumlich und zeitlich über mehrere Atemzüge gleichmäßig verteilt an. Die oben genannte Methode ist insofern nicht integrierend.
- Obwohl nur eine Stichprobe entnommen wird, ist der Probenbehälter mit 50 ml immer noch relativ groß. Dies verursacht räumliche Schwierigkeiten, wenn pro Patient z.B. acht Proben genommen und gleichzeitig Proben für mehrere Patienten versendet und gelagert werden müssen.
- Das System verlangt flüssigen Stickstoff, der üblicherweise im medizinischen Laborbetrieb nicht zur Verfügung steht und daher gesondert bewirtschaftet werden muß. Es fallen zusätzliche Betriebskosten an.
- Das Kühlfallen-System erfordert sorgfältige Wartung und ist schwer zu automatisieren. Eisbildung an verschiedenen Stellen macht es störanfällig. Bei Ausfall steht das gesamte Analysesystem still.
- Das Kühlfallen-System ist teuer in der Anschaffung.
- Der Zeitbedarf für eine Probenpräparation ist erheblich.

Grundsätzlich gibt es auch noch andere Möglichkeiten, Gasgemische aufzubereiten. So ist z.B. aus der US-A-4,327,575 eine Vorrichtung bekannt, bei der ein Gasgemisch nacheinander zwei Adsorptionsmittelschüttungen durchströmt. In der zunächst durchströmten Schüttung werden die für die Analyse nicht interessanten Gaskomponenten adsorbiert, in der nächsten Schüttung finden sich dann die zu analysierenden Gaskomponenten in angereicherter Form vor. Die aus der US-A-4,327,575 bekannte Vorrichtung dient in erster Linie zur Bestimmung von giftigen gasförmigen Substanzen und soll helfen, insbesondere die Bedingungen am Arbeitsplatz sicherer zu gestalten. Der Einsatz und die Umrüstung derartiger Vorrichtungen für die Überprüfung ausgeatmeter Atemproben ist bisher nicht angedacht worden.

Ausgehend von den oben geschilderten Problemen ist es eine Aufgabe der Erfindung, ein Verfahren zur Bestimmung des Isotopenverhältnisses im ausgeatmeten CO₂ einer Atemprobe zu schaffen, das mit geringem Aufwand durchgeführt werden kann und das darüber hinaus eine besonders genaue und sichere Analyse ermöglicht. Eine weitere Aufgabe der Erfindung besteht darin, eine Einrichtung zur Durchführung des Verfahrens bereitzustellen.

Gelöst werden diese Aufgaben mittels eines Verfahrens, das die Merkmale des Anspruches 1 aufweist und mittels einer Einrichtung, die die Merkmale des Anspruches 6 aufweist.

Vorteilhafte Weiterbildungen sind in den Unteransprüchen 2 bis 5 und 7 bis 14 angegeben.

Nach Anspruch 1 ist ein Verfahren vorgesehen, bei dem die Atemluft nacheinander durch mindestens eine wasserdampfadsorbierende und eine CO₂-adsorbierende Adsorptionsmittelschüttung geblasen wird, die CO₂ enthaltene Schüttung von einem Probenahmeort zu einem Untersuchungsort transportiert und dort durch Aufheizen und /oder Verringerung des Druckes das adsorbierte CO₂ desorbiert und auf seine Isotopenverhältnisse analysiert wird. Im Zuge der Desorption wird die CO₂-enthaltende Schüttung in einem Vorschritt zunächst auf einen Druck von ca. 20 mbar ausgepumpt und das dabei erhaltene Desorbat verworfen. Erst das im Zuge der weiteren sich an diesen Vorschritt anschließenden Desorption freigesetzte Desorbat wird dann der Isotopenanalyse zugeführt. Verfahrensgemäß wird damit erreicht, daß aus der zu analysierenden Adsorptionsmittelschüttung ein Großteil der möglicherweise störenden Verunreinigungen vor der Analyse entfernt wird. Man erhält auf diese Weise ein Analysedesorbat mit überraschend hohem Reinheitsgrad.

Im folgenden soll das erfindungsgemäße Verfahren allgemein erläutert werden. Mit Gasgemisch ist in diesem Zusammenhang immer jeweils eine von einem Patienten stammende Atemluftprobe gemeint.

Das erfindungsgemäße Verfahren ist insbesondere dann vorteilhaft, wenn wie hier die ungefähre Zusammensetzung eines Gasgemisches nach Substanzklassen vorbekannt ist und die Auftrennung des Gasgemisches den anschließenden Nachweis oder die anschließende Analyse erleichtert oder überhaupt erst ermöglicht. Hier wird eine besondere Gaskomponente zur weiteren Analyse, nämlich zur Bestimmung von Isotopenverhältnissen, abgetrennt.

Die Abtrennung einer schwach adsorbierten Komponente aus einem Gemisch mit stärker adsorbierten Komponenten durch eine Folge von zwei Schüttungen von Adsorptionsmitteln ist selbst dann möglich, wenn diese aus dem gleichen Material bestehen und Druck und Temperatur gleich sind. So ist zweckmäßig die zuerst durchströmte Schüttung im Verhältnis zur Menge des Gasgemisches so zu dimensionieren, daß nur die schwach adsorbierte Komponente in die zweite Schüttung überströmt. In der zuerst durchströmten Schüttung werden mehr oder weniger alle Komponenten adsorbiert, die abzutrennende Komponente jedoch nur zu einem geringen Teil. Diese wird hingegen in der zweiten Schüttung adsorbiert und liegt dort in reiner Form vor.

Im allgemeinen stört die durchbrechende, einer Schüttung zugeordnete Gaskomponente die Adsorption in der nachfolgenden Schüttung durch Verdrängungsdesorption. Ein Teil der bereits in der Schüttung adsorbierten Gaskomponente wird durch die der vorhergehenden Schüttung zugeordnete Gaskomponente ersetzt, wodurch die beabsichtigte Trennung der Gaskomponenten zum Teil aufgehoben wird. Man kann daher sinnvollerweise die einzelnen Schüttungen der Adsorptionsmittel im Verhältnis zur Menge und ungefähren Zusammensetzung des Gasgemisches und zur Aufnahmekapazität bei den in den Schüttungen herrschenden Druck- und Temperaturbedingungen so bemessen, daß die einer Schüttung zugeordnete Gaskomponente nicht in die nachfolgende Schüttung durchbricht. Die Gasmenge ist beispielsweise durch Druck und Volumen eines Vorratsgefäßes bestimmt, aus welchem das Gasgemisch in die Folge von Schüttungen eingeleitet wird. Aus der ungefähren Kenntnis der Zusammensetzung des Gasgemisches und den aus der Literatur bekannten Adsorptionsisothermen der Adsorptionsmittel folgt dann die minimal erforderliche Größe der einzelnen Schüttungen. Im allgemeinen wird mindestens eine Gaskomponente nicht oder nur in geringem Maße von irgendeiner der Schüttungen adsorbiert. Diese fungiert dann gleichsam als Trägergas für die anderen Komponenten durch alle Schüttungen hindurch.

Für die anschließende, der Analyse vorangehende Desorption ist eine möglichst hohe Sättigung des Adsorptionsmittels mit der ihm zugeordneten Gaskomponente erwünscht. Die Menge des je Masseneinheit des Adsorptionsmittels adsorbierten Gases wird von Druck und Temperatur bestimmt. Die Werte für die meisten praktisch vorkommenden Gase und Adsorptionsmittel sind in Gestalt der Adsorptionsisothermen der Literatur zu entnehmen. Die Wahl der optimalen Druck- und Temperaturwerte richtet sich nach den Adsorptionsisothermen der verschiedenen Adsorptionsmittel für die zu speichernden Gaskomponenten. Druck, Temperatur, Adsorptionsmittel und Größe der Schüttung werden so gewählt, daß das Durchbrechen einer Gaskomponente aus der ihr zugeordneten Schüttung in die nachfolgende unterbleibt und gleichzeitig eine möglichst hohe Sättigung erreicht wird.

Falls der erwünschte Trenneffekt unter Normalbedingungen von 1 bar und 20°C nicht ausreicht, werden Druck und/oder Temperatur entsprechend den Adsorptionsisothermen so gewählt, daß der Unterschied zwischen der stark adsorbierten Gasmenge der einen Art und der schwach adsorbierten Gasmenge der anderen Art möglichst groß ausfällt.

Dieser Unterschied wird besonders groß, wenn über den verschiedenen Schüttungen unterschiedliche Druck- und Temperaturbedingungen herrschen. Dann verhalten sich nämlich an sich identische Materialien wie verschiedene Adsorptionsmittel. Daher werden Druck und/oder Temperatur über Schüttungen aus identischen Adsorptionsmitteln so gewählt, daß der niedrigere Druck, beziehungsweise die höhere Temperatur vorangehen.

Für die Durchführung des erfindungsgemäßen Verfahrens werden Schüttungen vorzugsweise aus verschiedenen Adsorptionsmitteln vorgeschlagen. Die Schüttungen werden aus solchen Adsorptionsmitteln angelegt, welche einen Teil der im zu analysierenden Gasgemisch enthaltenen Komponenten stark, den anderen Teil aber nur schwach oder nicht adsorbieren. Bevorzugt werden solche Adsorptionsmittel, bei denen dieser Unterschied bereits unter Normalbedingungen von 20°C und 1 bar groß ist.

Für das erfindungsgemäß vorgeschlagene Verfahren eignen sich besonders zeolithische und Kohlenstoff-Molekularsiebe. Die zeolithischen Molekularsiebe sind fast universell anwendbar. Die Adsorptionseigenschaften der Molekularsiebe beruhen auf der großen inneren Oberfläche, auf hohen elektrostatischen Anziehungskräften und auf einem Siebeffekt im molekularen Maßstab. Sie lassen sich analog zu mechanischen Schüttsieben in eine Reihe mit wachsender Maschengröße ordnen, derart, daß jedem Gasmolekül eine bestimmte minimale Maschengröße zugeordnet ist, in welches es gerade noch hineinpasst; von Molekularsieben mit kleinerer Maschengröße kann es nicht adsorbiert werden. Die Molekularsiebtypen sind nach ihrer molekularen Maschengröße und ihrem Kristalltyp international einheitlich klassiert.

Die Schüttungen werden in der Reihenfolge durchströmt, daß das gröbere Molekularsieb dem feineren folgt; bei den zeolithischen Molekularsieben beispielsweise in der Reihenfolge der Typen 3A, 4A, 5A, 10X, 13X.

Um möglichst gute Analyseergebnisse zu erzielen, ist es erforderlich, einerseits hochgesättigte Adsorptionsmittel in die Desorptionsvorrichtung des Analysegerätes einzuschleusen, andererseits jedoch das unerwünschte Überströmen zu verhindern. In diesem Zusammenhang kann vorgesehen sein, daß mit Ausnahme der letzten Schüttung alle Schüttungen in einen ersten und in einen zweiten Teil geteilt werden, wovon der erste Teil entsprechend der Adsorptionskinetik gesättigt wird und der zweite Teil ungesättigt bleibt. Die zweiten Teile werden im Verhältnis zur Menge des zu analysierenden Gasgemisches so dimensioniert, daß die den Schüttungen zugeordneten Gaskomponenten nicht in die nachfolgenden Schüttungen durchbrechen. Bei der letzten Schüttung in einer Reihe von Schüttungen ist die Gefahr des Überströmens trivialerweise nicht gegeben. Bei qualitativen Analysen werden nur die ersten Teile in das Analysegerät eingebracht.

Weiterhin bezieht sich die Erfindung auf eine Einrichtung zur Durchführung des Verfahrens. Diese Einrichtung besteht aus zwei Einrichtungsteilen, von denen einer für die Adsorption und einer für die Desorption bestimmt ist. Wesentlich ist hier, daß am ersten Einrichtungsteil einsetz- und entfernbare, das jeweilige Adsorptionsmittel enthaltende Hülsen mit gasdurchlässigem Mantel und /oder gasdurchlässigen Endflächen angeordnet sind. Die Hülsen lassen sich aus dem ersten Einrichtungsteil entnehmen und in den zweiten zur Desorption dienenden Einrichtungsteil einsetzen. Der zweite Einrichtungsteil weist hierfür ein Hülsenmagaziniersystem zur Aufnahme der Hülsen auf, einen Probenüberträger und ein unter Vakuum und/oder Wärme setzbares Desorptionsgefäß, das seinerseits an das Einlaßsystem des Analysegerätes anschließbar ist.

Besonders vorteilhaft an dieser Einrichtung ist, daß die eigentliche Probenahme und die Analyse der Probe an unterschiedlichen Orten stattfinden können. Zur Entnahme einer Atemluftprobe wird nur der erste Einrichtungsteil benötigt, der in einer vorteilhaften Ausgestaltung gemäß Anspruch 9 trennbar von dem zweiten Einrichtungsteil vorgesehen werden kann. Nach Probenahme kann dem Einrichtungsteil die Hülse entnommen werden und diese dann zu dem zweiten Einrichtungsteil (der in aller Regel ein größeres stationäres Gerät ist) transportiert werden.

Weitere vorteilhafte Ausgestaltungen der Einrichtung sind in den Unteransprüchen 7 und 9 bis 14 beschrieben.

Die Erfindung soll im folgenden anhand der Analyse menschlicher oder tierischer Atemluftproben, näher erläutert werden.

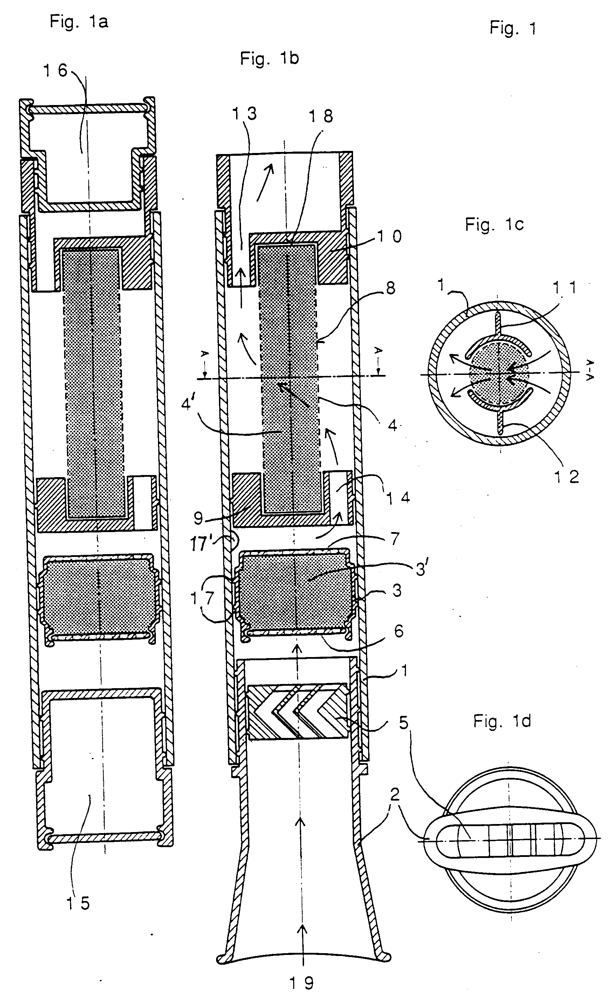

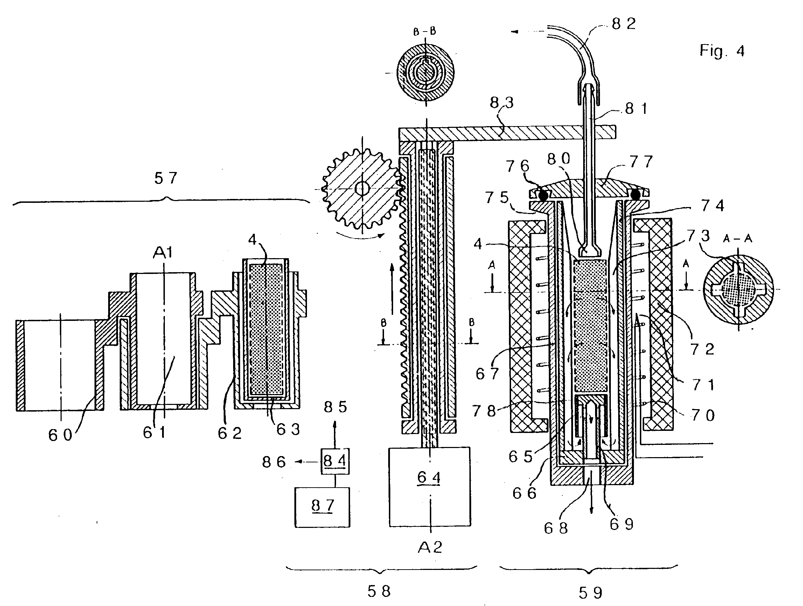

Die Fig.1 bis 6 stellen Teile einer erfindungsgemäßen Einrichtung zur Durchführung des erfindungsgemäßen Verfahrens dar. Die Fig.1 bis 3 beziehen sich auf die Adsorption als ersten Verfahrensschritt, die Fig.4 bis 6 auf die Desorption als zweiten Verfahrensschritt .

Die Einrichtungsteile nach den Fig.1 bis 3 dienen der Entnahme, Speicherung und Aufbereitung einer Atemprobe. Die Einrichtungsteile nach den Fig.4 bis 6 dienen der Bereitstellung und der Aufbereitung der zu analysierenden Atemgaskomponenten und sind einem geeigneten Analysegerät unmittelbar vorgeschaltet. Als geeignetes Analysegerät kann ein nach den nachfolgend, jedoch nicht ausschließlich genannten, an sich bekannten Verfahren arbeitendes Gerät verwendet werden: Flammenionisation, Massenspektroskopie, Gas-Chromatographie, optische Absorptionsspektroskopie, insbesondere Infrarot-Spektroskopie in den Ausführungsformen mit Laser als Lichtquelle und mit photoakustischem Nachweis oder in den Ausführungsformen als Differential-Absorptionsspektroskopie oder als Fourier-Transformations-Infrarot-Spektroskopie. Es werden insbesondere ein Massenspektrometer oder ein Fourier-Transformations-Infrarot-Spektrometer vorgeschlagen.

Die Einrichtungsteile nach den Fig.1 bis 3 sind Einheiten, die von den Einrichtungsteilen nach den Fig.4 bis 6 räumlich und zeitlich getrennt betrieben werden können. Durch die Unabhängigkeit von Probennahme und Analyse ist die Durchführung einer verteilten Probennahme möglich, das heißt, mehrere Probennahmestellen können einer Analysestation zuarbeiten. Das führt zu einer hohen, wirtschaftlich sinnvollen Auslastung der relativ aufwendigen Analysestation.

Fig.1 ist gegliedert in die Teilfiguren 1a, 1b, 1c und 1d. Fig.1a stellt als Ganzes eine Probennahmeröhre im Transport- und Lagerzustand, Fig. 1b dieselbe im Gebrauchszustand dar. Fig. 1c zeigt einen Querschnitt durch die Probennahmeröhre an der mit A-A bezeichneten Stelle, Fig.1d einen Blick auf die Röhre in Richtung des Pfeiles 19 in Fig. 1b.

Die für die Desorption im Einlaßsystem 127 des Analysegerätes vorgesehenen Adsorptionsmittel sind in gasdurchlässige Hülsen eingefüllt. Adsorptionsmittel liegen üblicherweise in Form von Pulvern oder kleinen, wenige Millimeter messenden Partikeln vor, welche sich schlecht handhaben lassen. Da die Desorptionstemperaturen Tröpfchenfalle 5, die das Eindringen größerer wasserhaltiger Tröpfchen in den stromabwärts gelegenen Teil verhindert. Dies ist insofern wichtig, als sonst die vorgeschlagene indirekte Atemluftmengenmessung verfälscht würde. Die in Fig.1b dargestellte Tröpfchenfalle 5 besteht aus einer Reihe V-förmiger Stege, die in Gebrauchsstellung senkrecht stehen und so angeordnet sind, daß eine direkte Durchsicht unmöglich ist. Die senkrechte Gebrauchsstellung wird durch die flache Formgebung des mundseitigen Endes und die anatomische Beschaffenheit des menschlichen Mundes nahegelegt. Das Mundstück 2 und die Tröpfchenfalle 5 sind zum Beispiel aus Polyäthylen hergestellt. Die Tröpfchenfalle 5 kann durchaus auch in anderer als der in Fig.1b beispielhaft dargestellten Form ausgebildet sein.

In Stromrichtung hinter der Tröpfchenfalle 5 befindet sich innerhalb der Hülse 3 eine Schüttung 3′ entweder aus dem preisgünstigen Adsorptionsmittel Silicagel oder aus zeolithischem Molekularsieb, vorzugsweise vom Typ 3A. Nach technischen Kriterien ist das Molekularsieb wegen seiner besseren Selektivität und besseren Adsorptionswirkung für Wasserdampf dem preisgünstigeren Silicagel vorzuziehen.

Die Hülse 3 ist zylinderförmig und hat mit gasdurchlässigen Öffnungen versehene, siebartige Endflächen 6 und 7. Die Hülse 3 besteht zum Beispiel aus Polyäthylen. Die Endfläche 6 ist als eindrückbarer Deckel ausgebildet, der nach Einfüllung der Schüttung 3′ eingesetzt wird.

Der Schüttung 3′ können einige an sich bekannte Indikatorkügelchen beigefügt werden, welche durch Farbumschlag den Grad der Wassersättigung anzeigen, oder die Schüttung kann ganz aus Indikatorkügelchen hergestellt werden. Die Indikatorkügelchen bestehen aus z. B. Molekularsiebkügelchen, welche mit Indikatorsubstanz durchsetzt sind. Durch Wahl transparenter Materialien für die Hülse 3 und das Tragrohr 1 ist das Adsorptionsmittel von außen sichtbar. Hierdurch wird der Farbumschlag, insbesondere zwischen den ebenfalls von außen sichtbaren, Markierungsringen 17 beobachtbar und die ungefähre Atemluftmengenmessung ermöglicht. Als Indikatorsubstanz wird Kobalt-II-Chlorid vorgeschlagen, welches durch Wasseraufnahme einen Hexaqua-Komplex bildet und dabei die Farbe von blau nach rosa wechselt.

Die Adsorption von Wasserdampf am Molekularsieb der Schüttung 3′ ist eine stark exotherme Reaktion. Wenn die Probennahme rasch erfolgt, kann die Reaktionswärme nicht an die Umgebung abgegeben werden und die durchströmende Atemluft erwärmt sich soweit, daß das in ihr enthaltene Kohlendioxid in der nachfolgenden Schüttung 4′ nur in geringem Maße adsorbiert wird. Für den Fall der raschen Probennahme wird daher vorgeschlagen, daß der erste Einrichtungsteil einen Temperaturindikator für die die Schüttung 3′ verlassende Atemluft, vorzugsweise einen durch Farbumschlag das Erreichen einer bestimmten Temperatur anzeigenden Farbfleck 17′ (Fig.1b) aus einer an sich bekannten Indikatorfarbe aufweist, wobei der Farbumschlag vorzugsweise bei einer Temperatur erfolgt, bei der das bereits in der Schüttung 4′ adsorbierte Kohlendioxid noch nicht desorbiert wird. Die Probennahme wird dann im Augenblick des Farbumschlages abgeschlossen.

Eine genauere Atemluftmengenmessung wird durch den erfindungsgemäß vorgeschlagenen, vorzugsweise als Flachbeutel aus Polyäthylen ausgebildeten, Beutel zum Auffangen der durchgeströmten Atemluft ermöglicht, welcher anstelle des Verschlußstopfens 16 in das Teil 10 eingesetzt wird.

Die Hülse 4 hat vorzugsweise die Form eines Zylinders mit einem Länge/Durchmesser-Verhältnis von mindestens 2:1. Die Mantelfläche 8 ist mit Ausnahme des oberen und unteren Randes möglichst ganz mit kleinen oder schmalen Öffnungen beliebiger Gestalt (z.B. Schlitze, kreisrunde Löcher, Langlöcher, schuppenartige Öffnungen) versehen, derart, daß Kugeln von ca. 0.8 mm Durchmesser nicht aus der Hülse entweichen können. Die für die Desorption vorgesehene Hülse 4 besitzt mindestens eine glatte Stirnfläche 18, an welcher ein Vakuumsaugnapf 80 (Fig.4) angreifen kann. Die Hülse 4 besteht vorzugsweise aus Reinaluminium oder Reinnickel, die Schüttung 4′ aus zeolithischem Molekularsieb.

Die innerhalb der Hülse 4 befindliche Schüttung 4′ aus Molekularsieb wird quer zur Zylinderachse durchströmt. Dies hat den Vorteil eines geringen Strömungswiderstandes sowohl für den Adsorptionsvorgang als auch für den späteren Desorptionsvorgang. Die Querströmung wird durch eine geeignete Unterteilung des Tragrohrvolumens bewirkt. Der von den Querschottwänden 9 und 10 begrenzte Tragrohrabschnitt wird von den Längsschottwänden 11 und 12 in zwei gegeneinander dichte Längshälften geteilt. Die Querschottwände 9 und 10 haben Gasdurchgangslöcher 13 und 14 sowie Aufnahmebohrungen für die Hülse 4. Die Längsschottwände 11 und 12 haben schalenförmige Erweiterungen, die an der Mantelfläche 8 der Hülse 4 außerhalb der Zustrom- und Abstrombereiche formschlüssig anliegen. Das gesamte Gebilde bestehend aus den Teilen 4, 9, 10, 11 und 12 stellt gleichsam einen Eindrückstopfen dar, der leicht aus dem Tragrohr 1 herausgezogen werden kann. Zur Entnahme der Hülse 4 kann es ohne Werkzeuge zerlegt werden.

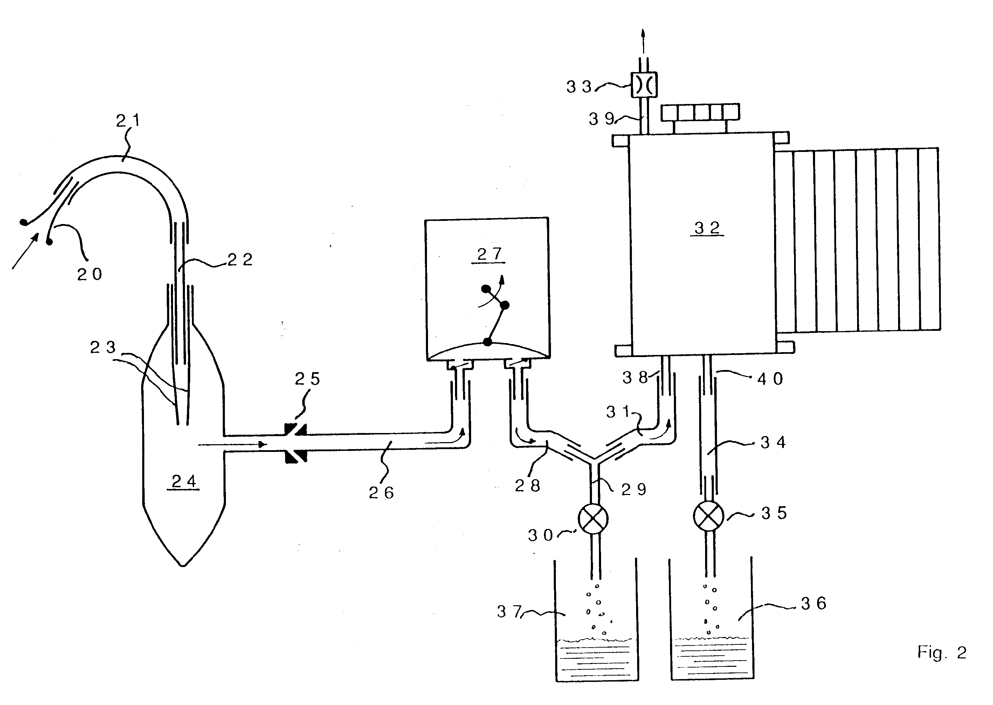

In Fig.2 ist eine erfindungsgemäße Einrichtung schematisch dargestellt. Über das Mundstück 20, den Verbindungsschlauch 21 und das Schlauchanschlußrohr 22 wird der Flachbeutel 24 mit Atemluft aufgefüllt. Während des Aufblasens verhindert ein aus zwei Flatterfolien 23 bestehendes Rückschlagventil das Zurückströmen der Atemluft. Der ca. 5 l fassende Flachbeutel 24 ist über die zum Austausch leicht lösbare Konusverbindung 25 und den Verbindungsschlauch 26 an den Membrankompressor 27 angeschlossen. Der Membrankompressor 27 ist mit seinem Kopf nach unten aufgehängt, damit das sich aus der Atemluft abscheidende Kondenswasser über den druckseitigen Verbindungsschlauch 28 abfließen kann. An der Y-Verzweigung 29 trennt sich das Kondenswasser von der komprimierten Luft, welche über den Verbindungsschlauch 31 zur Druckzelle 32 geleitet wird. Über das Ventil 30 wird das Wasser von Zeit zu Zeit in das Auffanggefäß 37 abgelassen. Mit der Drossel 33 werden Durchfluß und Druck eingestellt. Ein nicht dargestellter Zeitschalter bestimmt die Einschaltdauer des Membrankompressors 27 und somit die geförderte Luftmenge.

Die Teile 27, 32, 33 bestehen überwiegend aus metallischen Werkstoffen. Alle übrigen Teile können beispielsweise aus Polyäthylen hergestellt werden. Das Schlauchanschlußrohr 22 besteht beispielsweise aus Polyäthylen, hat ca. 10 mm Durchmesser und 1 mm Wandstärke, und ist mit den aus Polyäthylenfolie bestehenden Teilen 23 und 24 thermoplastisch verschweißt.

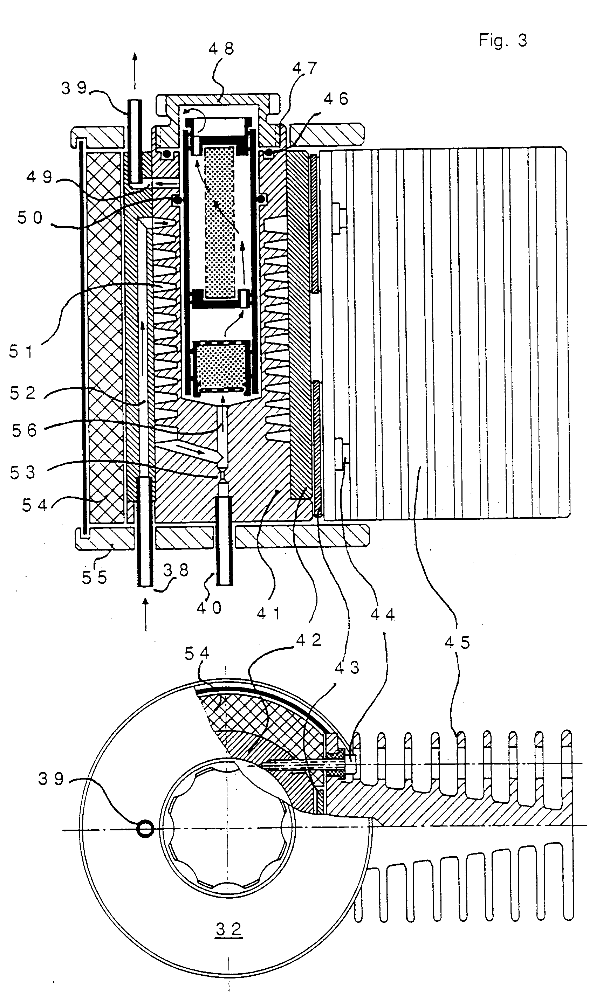

Die Druckzelle 32 ist in Fig.3 detailliert dargestellt. Der äußere Zylinder 42 umschließt dicht den mit einem Gewinde versehenen inneren Zylinder 41, wodurch sich der wendelförmige Kühlkanal 51 bildet. Im äußeren Zylinder befinden sich der Zuluftkanal 52, der über den Zuluftstutzen 38 mit dem Verbindungsschlauch 31 an den Membrankompressor 27 angeschlossen ist, sowie der Abluftkanal 49, welcher in den Abluftstutzen 39 mündet. Die Schrauben 44 spannen den äußeren Zylinder 42, die Peltier-Elemente 43 und den Kühlkörper 45 zusammen. Die Wärmeisolationsschichten 54 und 55 erhöhen das Temperaturgefälle zwischen der Umgebung und dem äußeren Zylinder 42, während die Peltier-Elemente 43 den Wärmestrom vom äußeren Zylinder 42 zum Kühlkörper 45 unterhalten.

Der innere Zylinder 41 besitzt eine zentrale Bohrung zur Aufnahme des Tragrohres 1 entsprechend Fig.1b einschließlich der in ihm enthaltenen, oben beschriebenen Teile, jedoch ohne das Mundstück 2. Die Füllung der Hülse 4 kann aus Kohlenstoff-Molekularsieb bestehen. Das Tragrohr 1 mit Inhalt wird durch die Öffnung des Schraubverschlusses 48, dessen Gewinde mit 47 bezeichnet ist, in die zentrale Bohrung des inneren Zylinders 41 gebracht oder aus ihr entnommen. Der O-Ring 46 dient der Abdichtung zum Zwecke des Druckaufbaus im gesamten System ab dem Membrankompressor 27 bis zur Drossel 33. Die O-Ringdichtung 50 verhindert einen Strömungskurzschluß zwischen der Einströmöffnung 56 und dem Abluftkanal 49 und erzwingt die Durchströmung der Hülsen 3 und 4 in Richtung der Pfeile. Über die Kapillare 53 kann das im Kühlkanal 51 kondensierende Wasser in den Abflußstutzen 40 fließen, aus welchem es über den Verbindungsschlauch 34 und das Ventil 35 von Zeit zu Zeit in das Sammelgefäß 36 entleert wird.

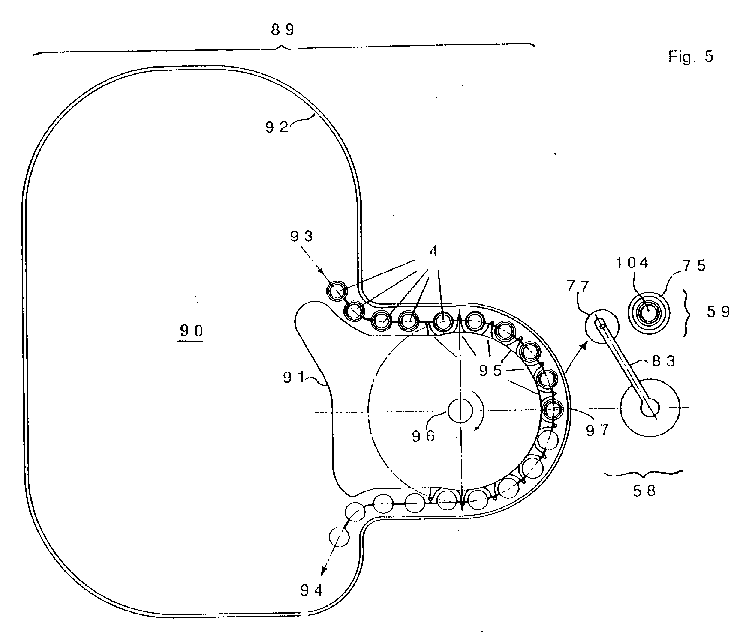

Fig.4 zeigt den aus zwei Gliedern 60 und 62 bestehenden Abschnitt einer Probenförderkette 57, einen Probenübertrager 58 und eine Desorptionsvorrichtung 59. Wie in Fig.5 dargestellt, werden die mit den Gasproben beladenen Hülsen 4 der Reihe nach mit dem Probenübertrager 58 in die Desorptionsvorrichtung 59 gebracht. Bis zu einige hundert Kettenglieder können aneinander gereiht werden, was die Beschickung der Probenförderkette mit entsprechend vielen Probenhülsen erlaubt.

Die ineinander gesteckten Kettenglieder 60 und 62 haben eine gemeinsame Drehachse A1. Der zylindrische Hohlraum 61 kann ein zylindrisches Sekundärgefäß 63 aufnehmen, welches eine mit einer Gasprobe beladene Hülse 4 enthält. Entsprechend kann das Sekundärgefäß 63, mit einem nicht dargestellten Stopfen gasdicht verschlossen, als Transportgefäß für die Probenhülse 4 zwischen dem Probennahmeort und dem Analysegerät dienen. Der Arm 83 des Probenübertragers 58 führt eine Hubbewegung und eine Drehbewegung um die Achse A2 mit Hilfe an sich bekannter, aus der Fig.4 ersichtlicher Antriebselemente aus. Der Motor für die Drehbewegung ist mit 64 bezeichnet.

Der Probenübertrager 58 besitzt einen Arm 83 mit einem Vakuumgreifer. Dieser besteht beispielsweise aus einem Röhrchen 81, das an seinem unteren Ende eine saugnapfartige Erweiterung 80 aufweist. Das andere Ende des Röhrchens 81 ist über den Schlauch 82 mit dem Eingang 85 des Ventils 84 verbunden, welches seinerseits an der Vakuumpumpe 87 angeschlossen ist. Mit dem Ventil 84 wird das Vakuum während des eigentlichen Übertragungsvorganges auf den Vakuumgreifer aufgeschaltet bzw. zum Loslassen der Hülse 4 durch Belüften über den Eingang 86 abgeschaltet. Der Übertrager 58 besitzt auch einen Arm 83, an welchem ein Deckel 77 mit Dichtring 76 zum vakuumdichten Verschliessen des Desorptionsgefäßes 67 befestigt ist. Der Deckel 77 muß aber nicht notwendigerweise eine Baueinheit mit dem Vakuumgreifer 80/81 bilden, wie in Fig.4 dargestellt.

Die Desorptionsvorrichtung 59 besteht aus dem Vakuumgefäß 67, dem Gefäßeinsatz 74 und der Heizeinrichtung 70/71/72. Das Vakuumgefäß 67 besteht aus einem Rohr, an dessen oberem Ende der Flansch 75 und an dessen unterem Ende die Rohranschlußöffnung 68 zum Anschluß an das Einlaßsystem 127 des Analysegerätes sitzen. Das Vakuumgefäß 67 ist in dem in Fig.4 dargestellten Ausführungsbeispiel außen von der Heizwicklung 70, dem Temperaturfühler 71 und dem Wärmeisolationsmantel 72 umgeben. In einer anderen Ausführung kann die Heizeinrichtung im Innern des Vakuumgefäßes 67 liegen.

Der Gefäßeinsatz 74 besteht aus einem Rohr, dessen unteres Ende von der Platte 66 abgeschlossen wird. Die Innenwand des Gefäßeinsatzes 74 ist mit Längsnuten 73 versehen, durch welche das desorbierte Gas abströmen kann. Der Gefäßeinsatz 74 kann zum Zwecke der Reinigung und Wartung leicht aus dem Vakuumgefäß 67 entnommen werden.

Die Rohranschlußöffnung 68 ist durch eine Vorrichtung gegen das Eindringen herabfallender Partikel, z. B. Molekularsiebpartikel, geschützt. Entsprechend dem in Fig.4 dargestellten Ausführungsbeispiel besteht diese Schutzvorrichtung aus dem in der Platte 66 sitzenden Rohrstutzen 69, dessen obere Eintrittsöffnungen 65 von einer seitlich weit heruntergezogenen Kappe 78 abgedeckt werden. Die Schutzkappe 78 dient hier gleichzeitig als Auflage für die Probenhülse 4. In einer anderen Ausführungsform kann die Aufgabe, das Eindringen von Partikeln zu verhindern, durch einen Partikelfilter gelöst werden.

In Fig.5 ist das Hülsenmagaziniersystem 89 zusammen mit dem Probenübertrager 58 und der Desorptionseinrichtung 59 aus Fig.4 dargestellt. Im Ausführungsbeispiel besteht es aus einer Tischplatte 90, auf der bis zu einige hundert Probenhülsen 4 in entsprechend vielen Sekundärgefäßen 63 und Kettengliedern 60,62 vorgelegt werden können, einem Kettenrad 95/96 und den Führungselementen 91 und 92. 95 ist der Zahn und 96 ist die Achse des von einem nicht dargestellten Schrittmotor angetriebenen Kettenrades. Die Führungselemente 91 und 92 führen während der Rotation des Kettenrades die mit den Probenhülsen 4 beladenen Kettenglieder der Reihe nach an der Übergabeposition 97 vorbei.

Mit 93 ist der Ketteneinlauf, mit 94 der Kettenauslauf bezeichnet.

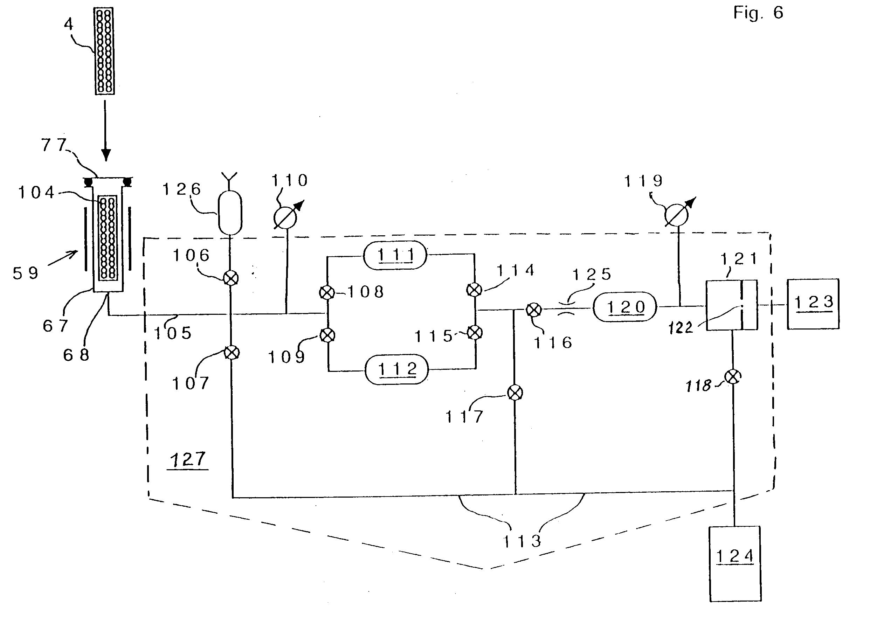

Der Einrichtungsteil nach Fig.6 stellt eine Desorptionsvorrichtung 59 mit einem an ein Analysegerät 123 angeschlossenen Einlaßsystem 127 dar. Als Analysegerät wird ein Instrument für die Isotopenverhältnismessung, insbesondere ein Massenspektrometer oder ein Fourier-Transformations-Infrarot-Spektrometer, vorgeschlagen. Das Massenspektrometer kann z.B. als magnetisches Sektorfeld-Instrument mit vorzugsweise mehreren Ionenauffängern für die Bestimmung von Isotopenverhältnissen oder alternativ als sogenanntes Quadrupol-Massenspektrometer ausgeführt sein.

Das Einlaßsystem 127 ist im wesentlichen ein zur Umgebung dicht abgeschlossenes System von Gasgefäßen, die über Rohrleitungen und Ventile miteinander verbunden sind. Das System kann zur Gänze oder in Abschnitten von einer Vakuumpumpe 124 ausgepumpt und von den Druckmeßgeräten 110 und 119 überwacht werden. Unter Druckmeßgeräten werden im folgenden auch Vakuummeßgeräte verstanden. Alle im folgenden genannten Druckwerte sind Absolutwerte. Unter Gasgefäßen werden im folgenden auch Rohrleitungsabschnitte verstanden, die von Ventilen abgeschlossen werden.

In Fig.6 enthält die Hülse 4 ein mit einer Gasprobe beladenes Adsorptionsmittel 4′; sie befindet sich außerhalb des gasdicht abgeschlossenen Systems. 104 ist eine ebensolche Hülse innerhalb des Desorptionsgefäßes 67, welches aus Fig.4 bekannt ist. An den Rohranschluß 68 ist das Verbindungsrohr 105 vom Desorptionsgefäß 67 zum übrigen System angeschlossen. Die Nummern 106, 107, 108, 109, 114, 115, 116, 117 und 118 benennen Ventile; 111, 112 und 120 sind Gasgefäße, die auch durch Rohrleitungsabschnitte gebildet werden können.

Die Druckreduzierstufe 121 vermindert den Druck des zu analysierenden Gases von dem relativ hohen Druck von ca. 100 mbar im Gefäß 120 auf den niedrigen Druck von ca. 0,0001 mbar in der Ionenquelle des Massenspektrometers 123, also um ca. 6 Größenordnungen. Diese Druckreduzierung wird durch einen hohen Strömungswiderstand 122, z.B. eine enge Öffnung in einer Goldfolie oder ein Plättchen aus dicht gesintertem Edelstahlpulver, erreicht. Wird an Stelle des im Ausführungsbeispiel genannten Massenspekrometers 123 ein anderes Analysegerät, beispielsweise ein Fourier-Transformations-Infrarot-Spektrometer, verwendet, so kann die Druckreduzierung andere Werte annehmen oder ganz entfallen.

Die Vakuumpumpe 124, z.B. eine Drehschieberpumpe, hat über die Pumpleitung 113 und die Ventile 107, 117 und 118 Zugriff zu verschiedenen Abschnitten des Einlaßsystems 127. Die Rohrleitungen bestehen beispielsweise aus Edelstahlrohren mit Innendurchmessern von ca. 5 mm bis 10 mm. Die Innenflächen sind vorzugsweise elektrolytisch poliert oder mit Teflon beschichtet, damit Verunreinigungen oder Reste vorangegangener Gasproben nicht an den Innenwänden haften bleiben. Aus dem gleichen Grund wird z.B. das gesamte in Fig.6 gezeigte System mit Ausnahme der Positionen 59, 123, 124, 126 und 113 durch Einbau in einen gestrichelt dargestellten, geheizten Kasten 127 auf einer Temperatur von vorzugsweise über 80°C gehalten. Zwecks eines schnellen Gaswechsels wird das Gaseinlaßsystem vorzugsweise im Laminarströmungsbereich bei Drücken von insbesondere über 1 mbar betrieben. Zur inneren Reinigung kann das gesamte System vom Filter 126 her mit reiner trockener Luft gespült werden, indem bei geschlossenem Deckel 77 und geschlossenen Ventilen 107 und 117 alle anderen Ventile geöffnet werden. Der Filter 126 kann beispielsweise aus einer Schüttung aus zeolithischem Molekularsieb und einer nachfolgenden Schüttung aus Aktivkohle bestehen.

Die Meßwertgeber für die Druckmeßgeräte 110 und 119 arbeiten beispielsweise mit piezoresistiven Elementen. Die Ventile 106, 107, 108, 109, 114, 115, 116, 117 und 118 können als elektromagnetische Ventile ausgebildet sein. Die zum zweiten Einrichtungsteil gehörenden elektrischen Versorgungsgeräte und elektronischen Kontrolleinheiten sind in an sich bekannter Weise ausgeführt. Die Steuerung der unten geschilderten Verfahrensschritte wird von einem Prozeßrechner unterstützt.

Die einzelnen erfindungsgemäßen Verfahrensschritte sollen nun anhand der oben beschriebenen Einrichtung und am Beispiel einer konkreten Diagnoseaufgabe geschildert werden, in welcher die Bestimmung des Isotopenverhältnisses im ausgeatmeten Kohlendioxid eines Patienten verlangt wird, dem eine diagnostische Droge verabreicht wurde.

Der Patient bläst seinen Atem so lange durch das Mundstück 2 (Fig.1b,1d) in die Schüttung 3′ aus Molekularsieb, bis etwa die Hälfte der Indikatorkügelchen einen Farbumschlag zeigt. Die Farbumschlagzone liegt dann zwischen den auf der Hülse 3 angebrachten Markierungsringen 17. Unter Normalbedingungen von 20°C und 1 bar wird in dieser Schüttung nur der in der ausgeatmeten Luft enthaltene Wasserdampf adsorbiert. Danach gelangt die Atemluft in die Schüttung 4′ aus Molekularsieb. In dieser Molekularsiebschüttung 4′ wird sowohl Wasserdampf als auch das in der Atemluft enthaltene Kohlendioxid adsorbiert. Wasserdampf kann bevorzugt adsorbiert werden und ist sogar imstande, bereits adsorbiertes Kohlendioxid zu verdrängen. Die der Schüttung 4′ vorgeschaltete Schüttung 3′ ist erfindungsgemäß aber so dimensioniert, daß sie den gesamten in der Atemluft enthaltenen Wasserdampf aufnehmen kann, bevor die Atemluft in die Schüttung 4′ eindringt.

Eine Atemluftmenge von 5 l wird von einer Person mit ca. 2 bis 4 Atemstößen aufgebracht. Diese Luftmenge enthält ca. 0,1 g Wasser und 0,4 g Kohlendioxid.Das Molekularsieb 3A kann unter Normalbedingungen etwa ein Fünftel seines Eigengewichtes an Wasser und das Molekularsieb etwa ein Zehntel seines Eigengewichtes an Kohlendioxid speichern. Eine Menge von ca. 1 g Molekularsieb 3A reicht also vollkommen aus, um 5 l Atemluft mit einer gewissen Sicherheitsreserve zu entwässern, ohne daß die Schüttung 3′ mit Wasser gesättigt ist. Die Menge der Schüttung 4′ wird erfindungsgemäß so dimensioniert, daß das Molekularsieb mit Kohlendioxid gut gesättigt ist, d.h., eine Menge von 2 g bis 3 g Molekularsieb für 5 l Atemluft sollte nicht überschritten werden. Die Sättigung wird indirekt durch Farbumschlag etwa der Hälfte der Indikatorkügelchen in der Hülse 3 angezeigt, welche auf den im Atem enthaltenen Wasserdampf ansprechen. Erfindungsgemäß ist die Schüttung 3′ so dimensioniert, daß sie nur zur Hälfte mit Wasserdampf gesättigt ist, wenn die Schüttung 4′ voll mit Kohlendioxid gesättigt ist.

Nachdem die Schüttung 4′ durch eine ausreichende Zahl von Atemstößen mit Kohlendioxid gesättigt ist, wird die Hülse 4 aus dem Einrichtungsteil nach Fig.1 entnommen und in ein Transport- bzw. Sekundärgefäß 63 nach Fig.4 gesteckt. Erfolgt die Analyse unmittelbar im Anschluß an die Probennahme, so wird das Sekundärgefäß 63 direkt in die Probenförderkette 57 geladen. Anderenfalls wird das Sekundärgefäß 63 bis zur Analyse mit einem Stopfen luftdicht verschlossen.

Der weitere Verlauf des Verfahrens wird anhand der Fig.4, 5 und 6 beschrieben. Die freigelegte Hülse 4 liegt innerhalb des Sekundärgefäßes 63. Die anderen Glieder der Förderkette 57 können ebenfalls in der gleichen Weise beladen sein. Die Hülse 4 wird, wie später der Reihe nach auch die übrigen Hülsen, an der Übergabeposition 97 vom Probenübertrager 58 übernommen und in die Desorptionsvorrichtung 59 übertragen.

Ziel des nächsten Verfahrensschrittes ist das Übertragen des in der Hülse 4 auf der Molekularsiebschüttung 4′ gespeicherten Gases unter größtmöglicher Erhaltung der Reinheit in das Gefäß 111, das von den Ventilen 108 und 114 abgeschlossen wird. Vor dem Füllen wird das Gefäß 111 über die geöffneten Ventile 114 und 117 und die Pumpleitung 113 von der ständig laufenden Pumpe 124 auf ein Vakuum von ungefähr 0,1 mbar Absolutwert ausgepumpt. Danach wird das Ventil 114 wieder geschlossen.

Bevor der Deckel 77 geöffnet werden kann, muß das Desorptionsgefäß 67 auf Umgebungsdruck geflutet werden. Dies wird durch Öffnen des Ventils 106 bei geschlossenen Ventilen 107, 108 und 109 erreicht. Der Druckausgleich findet über den Filter 126 und unter Überwachung durch das Druckmeßgerät 110 statt.

Nachdem das Druckmeßgerät 110 den Druckausgleich festgestellt hat, wird der Deckel 77 vom Desorptionsgefäß 67 abgehoben und die mit der Molekularsiebschüttung 4′ beladene Hülse 4 in das Desorptionsgefäß 67 eingebracht. Über das Ventil 107 und die Pumpleitung 113 wird das Desorptionsgefäß 67 mit der ständig laufenden Pumpe 124 verbunden und auf einen vorgegebenen Druck von ca. 20 mbar Absolutwert ausgepumpt. Zweck dieser Maßnahme ist das unmittelbare Abpumpen eventueller Verunreinigungen, bevor diese in die weiteren Teile des Einlaßsystems 127 gelangen.

Sobald der vorgegebene Druckwert erreicht ist, wird das Ventil 107 geschlossen und das Ventil 108 geöffnet. Durch Wärmeübertragung von der ständig auf ca. 100°C bis 200°C geheizten Wandung des Desorptionsgefäßes 67 auf die in der Hülse 104 befindliche Molekularsiebschüttung 4′ beginnt sich diese zu erwärmen. Folglich wird das auf dem Molekularsieb gespeicherte Gas desorbiert und durch das geöffnete Ventil 108 in das Gefäß 111 geleitet.

Nachdem der vom Druckmeßgerät 110 gemessene Druck auf einen vorbestimmten Wert von ca. 1 bar angestiegen ist, wird das Ventil 108 geschlossen. Das vorher auf dem in der Hülse 104 befindlichen Molekularsieb gespeicherte Gas ist nun zum überwiegenden Teil im Gasgefäß 111 zwischen den Ventilen 108 und 114 gefangen.

Im nächsten Verfahrensschritt wird das zu analysierende Gas aus dem Gefäß 111 in das Gefäß 120 übertragen, aus welchem das Gas über die Druckreduzierstufe 121 in die Ionenquelle des Massenspektrometers 123 gelangt. Das zwischen den Ventilen 116 und 118 augenblicklich gefangene Gas ruht während der Analyse, während der nur eine vernachlässigbar geringe Gasmenge verbraucht wird. Dieser Ruhezustand gewährleistet einen, insbesondere bei Präzisionsmessungen erforderlichen, konstanten Druck in der Meßzelle des Analyseinstruments.

Die Übertragung des Gases aus dem Gefäß 111 in das Gefäß 120 geschieht in folgenden Schritten. Zuerst wird das Volumen zwischen den Ventilen 116 und 118 bei geschlossenem Ventil 116 und geöffnetem Ventil 118 von der Pumpe 124 auf ca. 0,1 mbar ausgepumpt. Danach wird das Ventil 118 geschlossen, die Ventile 114 und 116 werden geöffnet. Das Gas beginnt nun aus dem Gefäß 111 in das Gefäß 120 überzuströmen, bis der vom Druckmeßgerät 119 festgestellte Druck einen vorbestimmten Wert von ca. 100 mbar erreicht hat, worauf beide Ventile 114 und 116 geschlossen werden. Die Drossel 125 verlangsamt den Überströmvorgang soweit, daß alle Schaltelemente zeitlich folgen können. Hiermit ist sichergestellt, daß im Gasgefäss 120 der vorbestimmte Druckwert innerhalb bestimmter Toleranzen eingestellt wird. Der im Gefäß 111 gespeicherte Gasvorrat reicht für wiederholte Füllungen des Gefäßes 120, so daß entsprechend oft Messungen des gleichen Gases wiederholt werden können.

Eine zweite Gasprobe wird dann aus einer zweiten Hülse 4 bzw. Hülse 104 in das Gasgefäß 112 übertragen. Dies geschieht analog der für die erste Probe geschilderten Weise, ebenso die Übertragung der zweiten Probe aus dem Gasgefäß 112 in das Gasgefäß 120, in welchem Fall die zweite Probe zur Messung gelangt. Auf diese Weise können die erste und die zweite Probe in einer sogenannten Probe/Referenz-Messung oder Probe/Standard-Messung unmittelbar miteinander verglichen werden.

Die Übertragung einer zweiten Gasprobe in das Gasgefäß 112 kann zeitgleich mit der Messung einer ersten Gasprobe erfolgen. Es können aufeinander folgend dritte, vierte, fünfte usw. Gasproben in das Gasgefäß 112 übertragen werden und alternierend mit der ersten Gasprobe gemessen werden. Auf diese Weise ist ein unmittelbarer Vergleich aller zweiten und folgenden Gasproben mit der ersten Gasprobe möglich.

Für die im Ausführungsbeispiel genannte Diagnoseaufgabe bedeutet dies,daß der zeitliche Verlauf des Isotopenverhältnisses im ausgeatmeten Kohlendioxid bestimmt werden kann; von der zu untersuchenden Person wird in bestimmten zeitlichen Abständen nach Verabreichung der Prüfsubstanz jeweils eine Atemprobe genommen.

Falls das Halten der ersten Gasprobe nicht erforderlich ist, können Gasproben alternierend in die Gasgefäße 111 und 112 übertragen werden oder es kann ganz auf das Gasgefäß 112 verzichtet werden.

## Patentansprüche

1. Verfahren zur Bestimmung des Isotopenverhältnisses im ausgeatmeten CO₂ einer Atemprobe, bei dem die Atemluft nacheinander durch mindestens eine Wasserdampf adsorbierende und eine CO₂-adsorbierende Adsorptionsmittelschüttung geblasen wird, die CO₂ enthaltende Adsorptionsmittelschüttung von einem Probenahmeort zu einem Untersuchungsort transportiert und dort durch Aufheizen und/oder Verringerung des Druckes das adsorbierte CO₂ desorbiert und auf seine Isotopenverhältnisse analysiert wird, wobei im Zuge der Desorption die CO₂ enthaltende Schüttung in einem Vorschritt zunächst auf einen Druck von ca. 20 mbar ausgepumpt und das dabei erhaltene Desorbat verworfen wird und erst das im Zuge der weiteren sich an diesen Vorschritt anschließenden Desorption freigesetzte Desorbat der Isotopenanalyse zugeführt wird.

2. Verfahren nach Anspruch 1, **dadurch gekennzeichnet**, daß die Analyse des Desorbats durch massenspektrometrische Trennung bzw. durch Infrarot-Spektroskopie, insbesondere mit photoakustischem Nachweis, erfolgt.

3. Verfahren nach einem der Ansprüche 1 und 2, **dadurch gekennzeichnet,** daß das Desorbat der Analyseeinrichtung im Laminarströmungsbereich bei Drücken über 1 mbar zugeführt wird.

4. Verfahren nach einem der Ansprüche 1 bis 3, **dadurch gekennzeichnet**, daß die zur Analyse bestimmte Adsorptionsmittelschüttung während des Transports vom Probeort zum Untersuchungsort in einem gasdicht verschlossenen Behälter aufbewahrt wird.

5. Verfahren nach einem der Ansprüche 1 bis 4, **dadurch gekennzeichnet**, daß beide Schüttungen aus gleichem Material bestehen.

6. Einrichtung zur Durchführung des Verfahrens nach einem der Ansprüche 1 bis 5, **dadurch gekennzeichnet**, daß sie aus einem ersten Einrichtungsteil für die Adsorption und einem zweiten Einrichtungsteil für die Desorption besteht, wobei im ersten Einrichtungsteil einsetz- und entfernbare, mindestens eine wasserdampfadsorbierende und eine CO₂-adsorbierende Adsorptionsmittelschüttung enthaltende Hülsen (3, 4) mit gasdurchlässigem Mantel (8) und/oder gasdurchlässigen Endflächen (7) angeordnet sind, während im zweiten Einrichtungsteil ein - vorzugsweise als Kettenförderer ausgebildetes - Hülsenmagaziniersystem (89) zur Aufnahme der Hülsen (4) mit den CO₂-adsorbierenden Adsorptionsmittelschüttungen, ein Pobenüberträger (58) und ein unter Vakuum und/oder Wärme setzbares Desorptionsgefäß (67) vorgesehen sind, das an das Einlaßsystem (127) eines Analysegerätes anschließbar ist, wobei das Einlaßsystem Mittel (107, 124) aufweist, um im Zuge der Desorption die CO₂-enthaltende Schüttung in einem Vorschritt zunächst auf einen Druck von ca. 20 mbar auszupumpen und das dabei erhaltene Desorbat verwerfen und wobei das Einlaßsystem (127) frei von solchen Baumaterialien ist, die bei den gewählten AnalysenVerfahren identische Signale wie die zu analysierenden Gaskomponenten abgeben.

7. Einrichtung nach Anspruch 6, **dadurch gekennzeichnet**, daß der erste Einrichtungsteil einen Gehäuseteil mit einer Lufteintritts- und einer Luftaustrittsöffnung aufweist, in dem in Strömungsrichtung gesehen zwischen der Lufteintritts- und der Luftaustrittsöffnung, die Hülsen (3,4) angeordnet sind, wobei die in der vorderen Hülse (3) angeordnete Adsorptionsmittelschüttung den in der Atemluftprobe enthaltenen Wasserdampf und die in der hinteren Hülse (4) angeordnete Schüttung das in der Atemluftprobe enthaltene CO₂ adsorbiert.

8. Einrichtung nach den Ansprüchen 6 und 7, **dadurch gekennzeichnet**, daß die Adsorptionsmittelschüttungen aus dem gleichen Material bestehen.

9. Einrichtung nach einem der Ansprüche 6 bis 8, **dadurch gekennzeichnet**, daß der erste Einrichtungsteil von der Einrichtung trennbar transportabel ausgebildet ist.

10. Einrichtung nach einem der Ansprüche 7 bis 9, **dadurch gekennzeichnet**, daß das Gehäuseteil als langgestrecktes zylindrisches Tragrohr ausgebildet ist, an dessen einem Längsende die Lufteintrittsöffnung vorgesehen ist.

11. Einrichtung nach Anspruch 10, **dadurch gekennzeichnet**, daß mindestens eine der die Adsorptionsmittelschüttungen aufnehmenden Hülsen mantelseitige Öffnungen besitzt und so in dem Tragrohr angeordnet ist, daß die Hülse quer zur Tragrohrachse durchströmt wird.

12. Einrichtung nach einem der Ansprüche 7 bis 11, **dadurch gekennzeichnet**, daß ein gasdichter Beutel mit seiner Einlaßöffnung an der Austrittsöffnung des Gehäuseteiles angeordnet ist.

13. Einrichtung nach einem der Ansprüche 6 bis 12, **dadurch gekennzeichnet**, daß als Adsorptionsmittelschüttungen zeolithische und/oder Kohlenstoff-Molekularsiebe vorgesehen sind.

14. Einrichtung nach einem der Ansprüche 6 bis 14, **dadurch gekennzeichnet,** daß die Adsorptionsmittelschüttungen in Strömungsrichtung eine zunehmende molekulare Maschengröße aufweisen.

## Claims

1. Method of determining the isotopic ratio in the exhaled CO₂ of a breath sample in which the exhaled air is blown successively through at least one water vapour-adsorbing adsorption agent filling and a CO₂-adsorbing adsorption agent filling, the adsorption agent filling containing CO₂ is transported from a sampling location to a testing location and the adsorbed CO₂ is desorbed there by heating and/or reducing the pressure and is analysed for its isotopic proportions, whereby in the course of the desorption the CO₂-containing filling is pumped out in a first step initially to a pressure of ca. 20 mbar and the desorbate thus obtained is discarded and only the desorbate liberated in the course of the further desorption following this first step is passed to the isotope analysis.

2. Method as claimed in Claim 1, characterised in that the analysis of the desorbate is effected by mass spectroscopic separation or by infrared spectroscopy, particularly with a photo-acoustic indication.

3. Method as claimed in one of Claims 1 and 2, characterised in that the desorbate is fed to the analysis device in the laminar flow range at pressures above 1 mbar.

4. Method as claimed in one of Claims 1 to 3, characterised in that the adsorption agent filling intended for analysis is stored during the transport from the sampling location to the testing location in a gas-tight closed container.

5. Method as claimed in one of Claims 1 to 4, characterised in that the two fillings comprise the same material.

6. Apparatus for carrying out the method as claimed in one of Claims 1 to 5, characterised in that it comprises a first apparatus portion for the adsorption and a second apparatus portion for the desorption, insertable and removable sleeves (3, 4) with a gas permeable wall (8) and/or gas permeable end surfaces (7) containing at least one water vapour-adsorbing and a CO₂-adsorbing adsorption agent filling being arranged in the first apparatus portion whilst provided in the second apparatus portion there are a sleeve magazine system (89) - preferably constructed as a chain conveyor - for receiving the sleeves (4) with the CO₂-adsorbing adsorption agent fillings, a sample transfer device (58) and a desorption vessel (67), which may be subjected to vacuum and/or heat and which may be connected to the inlet system (127) of an analysing device, whereby the inlet system has means (107, 124) to pump out the CO₂-containing filling in an initial step initially to a pressure of ca. 20 mbar in the course of the adsorption and to discard the desorbate thus obtained and whereby the inlet system (127) is free of those constructional materials which generate signals identical to those of the gas components to be analysed in the selected method of analysis.

7. Apparatus as claimed in Claim 6, characterised in that the first apparatus portion has a housing portion with an air inlet opening and an air outlet opening in which the sleeves (3, 4) are arranged between the air inlet opening and the air outlet opening, seen in the direction of flow, whereby the adsorption agent filling arranged in the front sleeve (3) adsorbs the water vapour contained in the exhaled air sample and the filling arranged in the rear sleeve (4) adsorbs the CO₂ contained in the exhaled air sample.

8. Apparatus as claimed in Claims 6 and 7, characterised in that the adsorption agent fillings comprise the same material.

9. Apparatus as claimed in one of Claims 6 to 8, characterised in that the first apparatus portion is constructed to be transportable separately from the apparatus.

10. Apparatus as claimed in one of Claims 7 to 9, characterised in that the housing portion is constructed as an elongate, cylindrical support tube at one of whose longitudinal ends the air inlet opening is provided.

11. Apparatus as claimed in Claim 10, characterised in that at least one of the sleeves accommodating the adsorption agent fillings has openings in its wall and is so arranged in the support tube that gas flows through the sleeve transverse to the support tube axis.

12. Apparatus as claimed in one of Claims 7 to 11, characterised in that a gas-tight bag is arranged with its inlet opening at the outlet opening of the housing portion.

13. Apparatus as claimed in one of Claims 6 to 12, characterised in that zeolitic and/or carbon molecular sieves are provided as the adsorption agent fillings.

14. Apparatus as claimed in one of Claims 6 to 14, characterised in that the adsorption agent fillings have an increasing molecular mesh size in the flow direction.

## Revendications

1. Procédé de détermination du rapport des isotopes dans le CO₂ expiré d'un échantillon d'air de respiration, dans lequel cet air est soufflé successivement à travers au moins une charge d'agent adsorbant la vapeur d'eau et une charge d'agent adsorbant le CO₂, la charge d'agent adsorbant contenant le CO₂ est transportée depuis un site de prélèvement d'échantillons vers un site d'analyse où le CO₂ adsorbé est désorbé par chauffage et/ou réduction de la pression et est analysé en vue d'établir le rapport de ses isotopes, la charge contenant le CO₂ étant tout d'abord mise par pompage dans une étape préalable sous un vide d'environ 20 mbars au cours de la désorption et le produit désorbé ainsi obtenu étant jeté, et le produit désorbé libéré au cours de la désorption ultérieure faisant suite à cette étape préalable étant seul envoyé à l'analyse des isotopes.

2. Procédé suivant la revendication 1, caractérisé en ce que l'analyse du produit désorbé est effectuée par séparation par spectrométrie de masse ou par spectroscopie infrarouge, notamment avec caractérisation photo-acoustique.

3. Procédé suivant l'une des revendications 1 et 2, caractérisé en ce que le produit désorbé est envoyé au dispositif d'analyse dans la zone d'écoulement laminaire à des pressions supérieures à 1 mbar.

4. Procédé suivant l'une des revendications 1 à 3, caractérisé en ce que la charge d'agent adsorbant destinée à l'analyse est conservée dans un récipient clos de façon étanche aux gaz pendant le transport du site de prélèvement au site d'analyse.

5. Procédé suivant l'une des revendications 1 à 4, caractérisé en ce que les deux charges sont constituées de la même matière.

6. Dispositif pour la mise en oeuvre du procédé suivant l'une des revendications 1 à 5, caractérisé en ce qu'il est constitué d'une première partie servant à l'adsorption et d'une seconde partie servant à la désorption, des douilles amovibles (3, 4) à enveloppe (8) perméable aux gaz et/ou à faces d'extrémités (7) perméables aux gaz, contenant au moins une charge d'agent adsorbant la vapeur d'eau et une charge d'agent adsorbant le CO₂ étant disposées dans la première partie, tandis qu'il est prévu dans la seconde partie un système (89) d'emmagasinage des douilles - de préférence réalisé comme transporteur à chaîne - destiné à recevoir les douilles (4) avec les charges d'agent adsorbant le CO₂, un système (58) de transfert des échantillons et un récipient de désorption (67) apte à être mis sous vide et/ou à recevoir de la chaleur, qui peut être raccordé au système d'admission (127) d'un appareil d'analyse, le système d'admission présentant des moyens (107, 124) permettant, dans une étape préalable au cours de la désorption, d'amener tout d'abord la charge contenant le CO₂ par pompage à une pression d'environ 20 mbars et de jeter le produit désorbé alors obtenu, et le système d'admission (127) étant dépourvu de matériaux de constitution qui délivrent, dans les méthodes analytiques choisies, des signaux identiques à ceux des composants du gaz à analyser.

7. Dispositif suivant la revendication 6, caractérisé en ce que sa première partie présente un boîtier doté d'une ouverture d'entrée d'air et d'une ouverture de sortie d'air dans lequel, vues dans la direction d'écoulement, les douilles (3, 4) sont disposées entre l'ouverture d'entrée et l'ouverture de sortie d'air, la charge d'agent adsorbant disposée dans la douille antérieure (3) adsorbant la vapeur d'eau contenue dans l'échantillon d'air de respiration et la charge disposée dans la douille postérieure (4) adsorbant le CO₂ contenu dans l'échantillon d'air de respiration.

8. Dispositif suivant les revendications 6 et 7, caractérisé en ce que les charges d'agent adsorbant sont constituées de la même matière.

9. Dispositif suivant l'une des revendications 6 à 8, caractérisé en ce que sa première partie est réalisée sous une forme transportable pouvant en être séparée.

10. Dispositif suivant l'une des revendications 7 à 9, caractérisé en ce que le boîtier est réalisé comme tube de support cylindrique allongé à l'une des extrémités longitudinales duquel l'ouverture d'entrée d'air est prévue.

11. Dispositif suivant la revendication 10, caractérisé en ce que l'une au moins des douilles recevant les charges d'agent adsorbant présente des ouvertures pratiquées dans l'enveloppe et est disposée dans le tube de support de manière que le courant y passe transversalement à l'axe du tube de support.

12. Dispositif suivant l'une des revendications 7 à 11, caractérisé en ce qu'une poche étanche aux gaz est disposée avec son ouverture d'accès contre l'ouverture de sortie du boîtier.

13. Dispositif suivant l'une des revendications 6 à 12, caractérisé en ce que des tamis moléculaires zéolitiques et/ou au carbone sont prévus comme charges d'agent adsorbant.

14. Dispositif suivant l'une des revendications 6 à 13, caractérisé en ce que les charges d'agent adsorbant présentent une grandeur de maille moléculaire qui croît dans la direction d'écoulement.
